# EUROPEAN PATENT SPECIFICATION

(11) **EP 2 894 583 B1**
(45) Date of publication and mention of the grant of the patent: **11.03.2020**
(21) Application number: 15151061.7
(22) Date of filing: 14.01.2015
(51) Int. Cl.: G16H 20/30, A63B 24/00

(54) **Safety ensuring system of exercise machine and method of ensuring safety while using an exercise machine**
Sicherheitsgewährleistungssystem einer Übungsmaschine und Verfahren zur Gewährleistung der Sicherheit bei der Verwendung einer Übungsmaschine
Système de sécurité pour une machine d'exercice et procédé permettant d'assurer la sécurité d'une machine d'exercice

(30) Priority: 14.01.2014 TW 103101324
(43) Date of publication of application: 15.07.2015
(73) Proprietor: Gee Hoo Fitec Corp, New Taipei City 244 (TW)
(72) Inventor: Hsu, Ching Lu, 112 Taipei City (TW)
(74) Representative: Viering, Jentschura & Partner mbB Patent- und Rechtsanwälte

(56) References cited:
- EP-A1- 1 369 147
- EP-A2- 0 199 442
- US-A1- 2013 253 943
- US-B1- 6 659 946

## Description

### BACKGROUND OF THE INVENTION

### 1. Technical Field

The present invention relates generally to safety at using an exercise machine, and more particularly, to a safety ensuring system of exercise machines and method of ensuring safety while using an exercise machine.

### 2. Description of Related Art

Lacking of exercise has become a typical problem for people in the modern world. According to related studies, exercising is good for cardiopulmonary function and blood circulation, which effectively lowers the chances of suffering from chronic diseases. Therefore, those who care about their health would try to make exercise a habit, and using exercise machines is a widely chosen way because it is unaffected by weather conditions.

Moderate amount of exercise is beneficial to health, but it is not easy for people to aware of their physiological state (for example, blood pressure, rhythm of heart, or hemoglobin oxygen saturation) during exercising. And doing exercise under the condition of abnormal physiological state could be harmful, or even leads to sudden death.

Take a gym for example, although there are various kinds of exercise machines provided for their members, and there might be fitness trainers aside, the physiological state of the members is still unlikely to be monitored. Prior art systems such as US 2013/0253943 or EP 0 199 442 monitor the health state of a user of exercise machines during the use of these machines. However, said prior art systems fail to prevent users who are in poor or critical health state from doing (intensive) exercise. Hence, the safety of using exercise machines is not able to be ensured effectively, and the users are actually exposed to some kind of potential hazard.

### BRIEF SUMMARY OF THE INVENTION

In view of the above, the primary objective of the present invention is to provide a safety ensuring system of exercise machine, and a method of ensuring safety while using an exercise machine, which could effectively ensure exercise safety.

The present invention provides a safety ensuring system of exercise machine as defined in independent claim 7.

The present invention further provides a method of ensuring safety while using an exercise machine as defined in independent claim 1.

Whereby, the present invention determines the permission for a user to use an exercise machine according to his/her physiological state, which prevents the user in poor physiological state from exercising, and therefore the safety of using an exercise machine is effectively ensured.

### BRIEF DESCRIPTION OF THE SEVERAL VIEWS OF THE DRAWINGS

The present invention will be best understood by referring to the following detailed description of some illustrative embodiments in conjunction with the accompanying drawings, in which
FIG. 1 is a block diagram of the safety ensuring system of a preferred embodiment of the present invention;
FIG. 2 is a block diagram of the physiological detection instrument of the preferred embodiment of the present invention;
FIG. 3 is a block diagram of the exercise machine of the preferred embodiment of the present invention; and
FIG. 4A and FIG. 4B are flow charts of the safety ensuring method of the preferred embodiment of the present invention.

### DETAILED DESCRIPTION OF THE INVENTION

The detailed description and technical contents of the present invention will be explained with reference to the accompanying drawings. However, the drawings are for illustration only and cannot be used to limit the present invention.

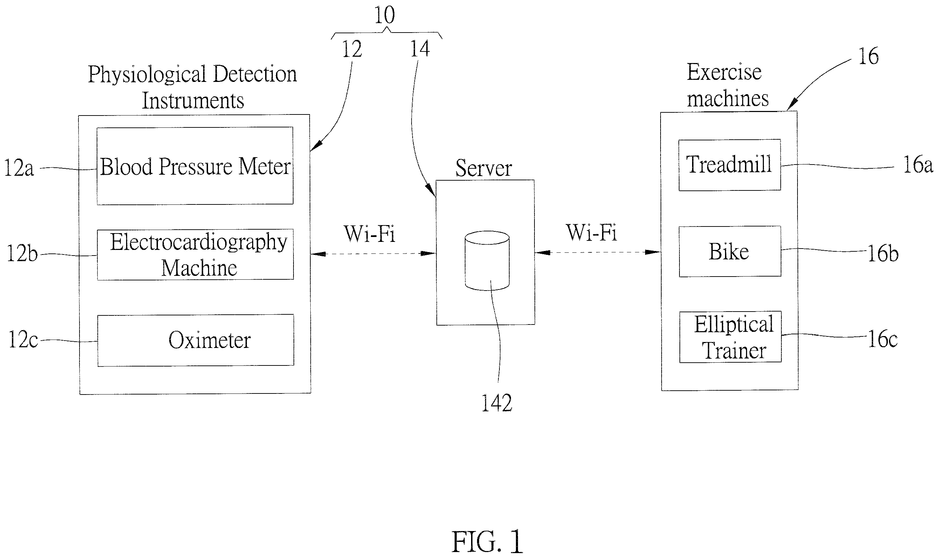

As shown in FIG. 1, a safety ensuring system of a preferred embodiment of the present invention includes a physiological detection device 10 and a plurality of exercise machines 16.

In the preferred embodiment, the safety ensuring system is applied in a gym, and each member of the gym is provided with an identifying device, which is a NFC (Near Field Communication) electronic tag in the preferred embodiment. Each NFC tag held by each member is built in with an identity code. Of course, other identifying devices (for example, member cards with barcodes) could be adopted in practice.

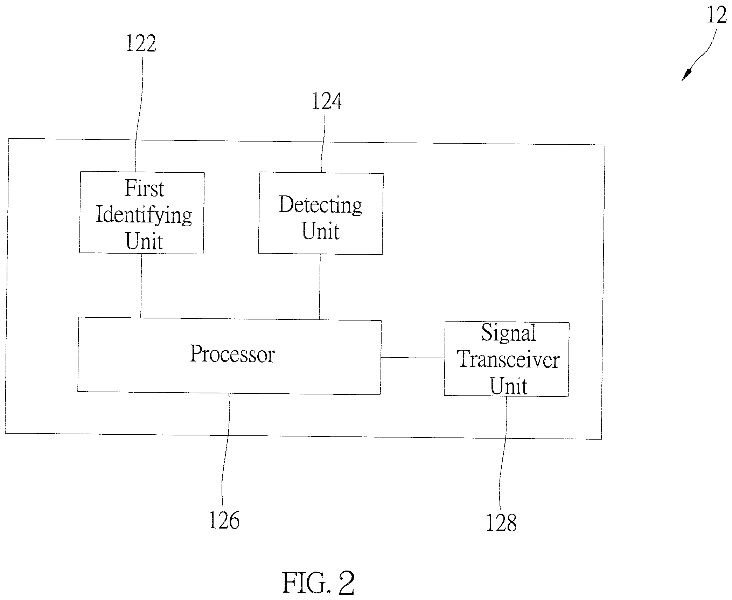

In the preferred embodiment, the physiological detection device 10 includes a plurality of physiological detection instruments 12 and a server 14. The physiological detection instruments 12 include a blood pressure meter 12a, an electrocardiography machine 12b, and an oximeter 12c. Other physiological detection instruments 12 could be adopted in other embodiments, such as body fat meter, non-invasive blood glucose meter, etc. As shown in FIG. 2, each physiological detection instrument 12 includes a first identifying unit 122, a detecting unit 124, a processor 126, and a signal transceiver unit 128. The first identifying unit 122 is a NFC reader, which communicates with the NFC tags held by the members to retrieve identity codes.

The detecting unit 124 detects physiological signals of human body. Take the blood pressure meter 12a for example, the detecting unit 124 detects blood pressure and pulse of human body, and transmits corresponding electrical signals to the processor 126 to generate corresponding physiological signals. Similarly, for the electrocardiography machine 12b, the detecting unit 124 thereof detects bioelectrical potentials of heartbeats, and then sends them to the processor 126. The detecting unit 124 of the oximeter 12c emits specific light onto human body, and receives the intensity of light that goes through human body by a sensor; after that, the processor 126 processes related measurements to obtain the physiological signals of the hemoglobin oxygen saturation.

The processor 126 sends out the physiological signals, including blood pressure, pulse, bioelectrical potentials of heartbeat, and the hemoglobin oxygen saturation etc., and the identity code identified by the first identifying unit 122 through the signal transceiver unit 128. In the preferred embodiment, the signal transceiver unit 128 transmits data with Wi-Fi signals. Bluetooth, RF, or other wireless transmission means could be selected in other embodiments. In the present embodiment, the physiological detection instruments 12 are medical grade instruments, which could provide accurate detecting results.

The server 14 receives the identity codes and the physiological signals from the signal transceiver units 128 of the physiological detection instruments 12. The server 14 has a data storage unit 142, which saves the identity codes and the corresponding physiological signals. In addition, the server is built in with a comparing program, which analyzes the received physiological signals to generate a physiological state. In the preferred embodiment, the physiological state could be classified as a normal condition, a critical condition, or an abnormal condition by definition. The analysis of the physiological state is based on the standards provided by World Health Organization (WHO) or health authorities at the local area of the gym. In more details, the normal condition indicates that the physiological signals are within a standard range, which is safe for doing exercise normally; the critical condition indicates that the physiological signals are outsight the standard range but not falling into a hazard range, which is suitable for doing exercise with limited intensity; the abnormal condition indicates that the physiological signals are falls into the hazard range, which is not recommended for doing exercise. Take blood pressure for example, the normal condition means that systolic pressure is between 100 and 139mmHg, and diastolic pressure is between 66 and 90mmHg; the critical condition means that systolic pressure is between 140 and 179mmHg, and diastolic pressure is between 91 and 100mmHg; the abnormal condition means that systolic pressure is higher than 180mmHg, and diastolic pressure is higher than 101mmHg. Similarly, for electrocardiography, the condition of the physiological state is determined by analyzing the graph itself; as for blood glucose, the value of blood glucose is the baseline for judgment, and so on.

After analyzing the physiological signals, the server 14 saves the physiological state (as normal, critical, or abnormal condition) which corresponds to the identity code into the data storage unit 142.

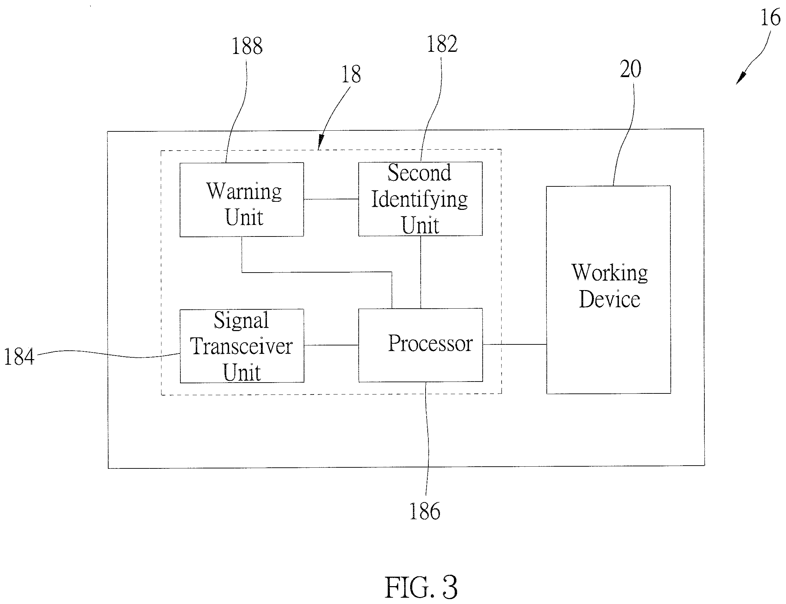

In the preferred embodiment, the exercise machines 16 include a treadmill 16a, a bike 16, and an elliptical trainer 16c. Other exercise machines, such as rowing machine, could be selected in other embodiments. As shown in FIG. 3, each exercise machine 16 includes a control device 18 and a working device 20. The control device 18 includes a second identifying unit 182, a signal transceiver unit 184, a processor 186, and a warning unit 188. The second identifying unit 182 is a NFC reader too, which reads the identity codes of the NFC tags and transmits them to the processor 186. The signal transceiver unit 184 is connected to the server 14 with Wi-Fi signals. In other embodiments, Bluetooth, RF, other wireless transmission means, or cables and the likes of which could be selected for signal transmission between the control device 18 and the server 14. The processor 186 not only turns on and off the working device 20, but also controls the operation intensity of the working device 20 according to the physiological state saved in the data storage unit 142. The processor 186 communicates with the server 14 through the signal transceiver unit 184 to retrieve data in the data storage unit 142. In the preferred embodiment, the warning unit 188 is a speaker, which is controlled by the processor 186 to sound an alarm. In practice, the warning unit 188 could be a stand alone monitor, or a monitor with a speaker.

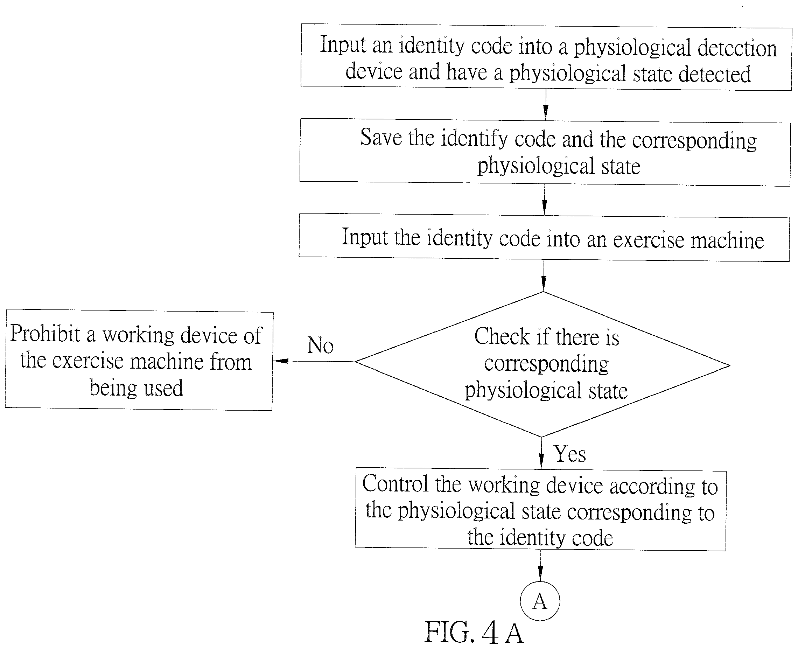

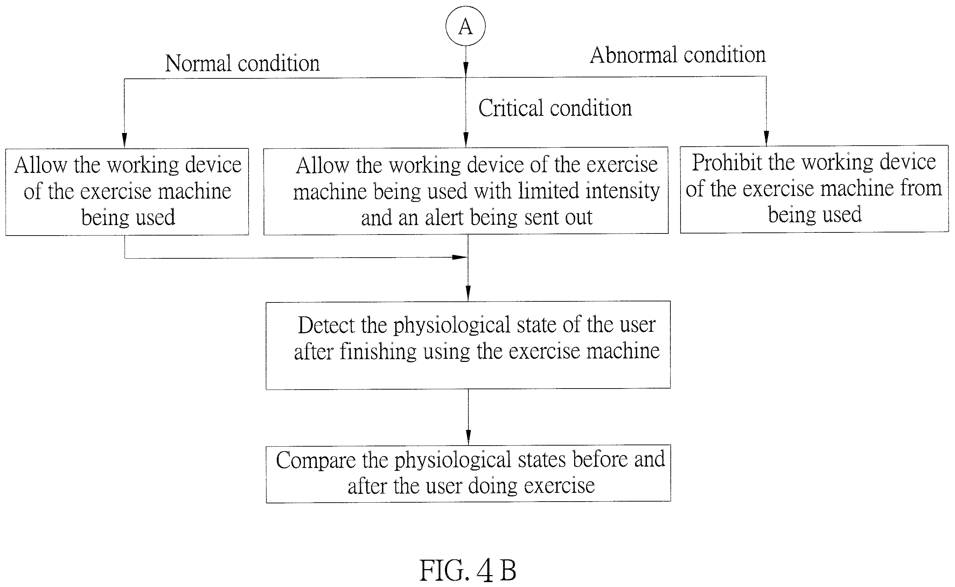

With the aforementioned system, a method of ensuring safety could be applied, as shown in FIG. 4A and 4B.

First of all, before the user using the exercise machine, the NFC tag held by the user is read by the first identifying unit 122 of the physiological detection instrument 12 to obtain the identity code. Next, the physiological detection instrument 12 detects physiological signals of the user. After detecting, the physiological detection instrument 12 transmits the identity code of the user and the physiological signals to the server 14 to be saved in the data storage unit 142. The server 14 executes the comparing program to analyze the physiological signals, and generates the physiological state (as normal, critical, or abnormal condition) to be saved in the data storage unit 142. Whereby, a correspondence between the identity code and the physiological state is established.

Then, the NFC tag of the user is read by the second identifying unit 182 of any one of the exercise machines 16, which the user intends to use, to obtain the identity code. The processor 186 then communicates with the server 14 through the signal transceiver unit 184 to retrieve the physiological state corresponding to the identity code.

If the processor 186 finds out that the identity code and the corresponding physiological state are not saved in the data storage unit 142, it means that the physiological state of the user has not yet been detected. In such case, the working device 20 is prohibited from being used. Furthermore, the warning unit 188 sends out an alert to request the user to have the physiological state detected.

If the identity code and the corresponding physiological state are found in the data storage unit 142, the processor 186 determined whether the working device 20 is allowed to be used according to the physiological state.

If the physiological state is classified as the normal condition, the working device 20 is allowed to be used, and the user is able to do exercise normally.

If the physiological state is classified as the critical condition, the working device 20 is allowed to be used with limited intensity. In this way, the user could only exercise moderately for safety. In addition, the warning unit 188 sends out another alert to remind the user not to do exercise too fiercely. For example, if the exercise machine 16 used by the user is the treadmill 16a, then an endless track of the working device 20 of the treadmill 16a is restricted from rotating higher than 4 km per hour, which allows the user to walk slowly on it.

If the physiological state is classified as the abnormal condition, the working device 20 is prohibited from being used, and the warning unit 188 sends out another alert to remind the user that he/she is not suitable for exercising right now, which ensures the safety of the user.

After doing exercise with any one of the exercise machines 16, the user could be reminded by the warning unit 188 to have the physiological state detected again. After detecting, the physiological signals and the physiological state at this time point is saved by the server 14 for comparison purpose. If the physiological state of the user is classified as the abnormal condition after doing exercise, the user could realize that he has to take certain appropriate actions, such as going to hospital or so. In practice, the server 14 could transmit comparing results back to the physiological detection instrument 12 to be displayed there or on another monitor instead. Whereby, the user is able to know his/her physiological states before and after doing exercise.

In summary, the safety ensuring system and the method of the present invention could determine the permissions for using exercise machines according to the physiological state of the user, which effectively prevents the user in poor physiological state from doing exercise, and increases the safety at using exercise machines.

It must be pointed out that the embodiments described above are only some preferred embodiments of the present invention.

## Claims

1. A computer-implemented method of ensuring safety which is applied to a safety ensuring system comprising a physiological detection device (10) and an exercise machine (16), wherein the physiological detection device (10) has a data storage unit (142), the physiological detection device (10) detects a physiological state of a human body and saves the physiological state in the data storage unit (142); the exercise machine (16) including a control device (18) and a working device (20), the control device (18) is electrically connected to the physiological detection device (10), the computer-implemented method of ensuring safety comprises the steps of:
A. establishing a correspondence between an identity code and a physiological state of a user;
B. inputting the identity code into the exercise machine (16); and
C. retrieving the physiological state corresponding to the inputted identity code;
**characterized in** the further step of:
D. determining if the exercise machine (16) is allowed to be used or not according to the retrieved physiological state;
wherein the exercise machine (16) is allowed to be used if the retrieved physiological state is classified as a normal condition; the exercise machine (16) is prohibited from being used if the retrieved physiological state is classified as an abnormal condition;
wherein the exercise machine (16) is allowed to be used with an alert being sent out if the physiological state is classified as a critical condition;
wherein the exercise machine (16) is allowed to be used with limited intensity if the physiological state is classified as a critical condition.

2. The method of claim 1, wherein the step A comprises the steps of:
inputting the identity code into the physiological detection device (10);
detecting the physiological state of the user with the physiological detection device (10); and
establishing a correspondence between the inputted identity code and the detected physiological state.

3. The method of claim 2, wherein the physiological detection device (10) detects physiological signals of human body; and the physiological state is obtained by analyzing the physiological signals.

4. The method of claim 3, wherein the physiological signals include blood pressure, hemoglobin oxygen saturation, or electrocardiography.

5. The method of claim 1, wherein in the step C, if the physiological state is not retrievable, the exercise machine (16) is prohibited from being used.

6. The method of claim 1, further comprising the steps of detecting another physiological state of the user after the user finishing using the exercise machine (16) to be analyzed.

7. A safety ensuring system of an exercise machine (16), which comprises a physiological detection device (10) and an exercise machine (16), wherein
the physiological detection device (10) has a data storage unit (142), wherein the physiological detection device (10) is configured to detect a physiological state of a human body and to save the physiological state in the data storage unit (142); and
the exercise machine (16) including a control device (18) and a working device (20), wherein the control device (18) is electrically connected to the physiological detection device (10); **characterized in that**
the control device (18) is configured to determine whether the working device (20) is allowed to be used according to the physiological state saved in the data storage unit (142);
wherein the control device (18) of the exercise machine (16) is configured to retrieve the physiological state saved in the data storage unit (142); the control device (18) is configured to control the working device (20) to be allowed to be used if the physiological state is classified as a normal condition; the control device (18) is configured to control the working device (20) to be prohibited from being used if the physiological state is classified as an abnormal condition.;
wherein the control device (18) of the exercise machine (16) includes a warning unit (188); the control device (18) is configured to control the working device (20) to be allowed to be used with an alert sent out by the warning unit (188) if the physiological state is classified as a critical condition;
wherein the control device (18) is configured to control the working device (20) to be allowed to be used with limited intensity if the physiological state is classified as a critical condition.

8. The safety ensuring system of claim 7, wherein the physiological detection device (10) includes a physiological detection instrument (12) and a server (14), wherein the physiological detection instrument (12) is configured to detect physiological signals of a human body; the server (14) is connected to the control device (18), and is configured to generate the physiological state by analyzing the physiological signals; the server (14) has the data storage unit (142).

9. The safety ensuring system of claim 8, wherein the physiological signals include blood pressure, hemoglobin oxygen saturation, or electrocardiography.

10. The safety ensuring system of claim 7, wherein the physiological detection device (10) includes a first identifying unit (122) to receive an identity code; the data storage unit (142) is saved with the identity code received by the first identifying unit (122) and the physiological state which corresponds to the identity code; the control device (18) includes a second identifying unit (182) to receive the identity code, the control device (18) is configured to retrieve the physiological state saved in the data storage unit (142) according to the identity code received by the second identifying unit (182).

## Patentansprüche

1. Ein computerimplementiertes Verfahren zum Sicherstellen der Sicherheit, das bei einem Sicherheits-Sicherstellungssystem angewendet wird, welches eine physiologische Detektionsvorrichtung (10) und ein Trainingsgerät (16) umfasst, wobei die physiologische Detektionsvorrichtung (10) eine Datenspeichereinheit (142) aufweist, die physiologische Detektionsvorrichtung (10) einen physiologischen Zustand eines menschlichen Körpers detektiert und den physiologischen Zustand in der Datenspeichereinheit (142) speichert; das Trainingsgerät (16) eine Steuervorrichtung (18) und eine Arbeitsvorrichtung (20) umfasst, die Steuervorrichtung (18) elektrisch mit der physiologischen Detektionsvorrichtung (10) verbunden ist, wobei das computerimplementierte Verfahren zum Sicherstellen der Sicherheit die folgenden Schritte umfasst:
A. Herstellen einer Übereinstimmung zwischen einem Identitätscode und einem physiologischen Zustand eines Benutzers;
B. Eingeben des Identitätscodes in das Trainingsgerät (16); und
C. Abrufen des physiologischen Zustands, der dem eingegebenen Identitätscode entspricht;
**gekennzeichnet durch** den folgenden weiteren Schritt:
D. Bestimmen, ob das Trainingsgerät (16) verwendet werden darf oder nicht, gemäß dem abgerufenen physiologischen Zustand;
wobei das Trainingsgerät (16) verwendet werden darf, wenn der abgerufene physiologische Zustand als ein normaler Zustand klassifiziert wird; das Trainingsgerät (16) nicht verwendet werden darf, wenn der abgerufene physiologische Zustand als ein unnormaler Zustand klassifiziert wird;
wobei das Trainingsgerät (16) mit einem Alarm, der ausgesendet wird, wenn der physiologische Zustand als ein kritischer Zustand klassifiziert wird, verwendet werden darf;
wobei das Trainingsgerät (16) mit begrenzter Intensität verwendet werden darf, wenn der physiologische Zustand als ein kritischer Zustand klassifiziert wird.

2. Das Verfahren nach Anspruch 1, wobei der Schritt A die folgenden Schritte umfasst:
Eingeben des Identitätscodes in die physiologische Detektionsvorrichtung (10);
Detektieren des physiologischen Zustands des Benutzers mit der physiologischen Detektionsvorrichtung (10); und
Herstellen einer Übereinstimmung zwischen dem eingegebenen Identitätscode und dem detektierten physiologischen Zustand.

3. Das Verfahren nach Anspruch 2, wobei die physiologische Detektionsvorrichtung (10) physiologische Signale des menschlichen Körpers detektiert und der physiologische Zustand durch Analysieren der physiologischen Signale erhalten wird.

4. Das Verfahren nach Anspruch 3, wobei die physiologischen Signale Blutdruck, Hämoglobin-Sauerstoffsättigung oder Elektrokardiographie umfassen.

5. Das Verfahren nach Anspruch 1, wobei im Schritt C, wenn der physiologische Zustand nicht abrufbar ist, das Trainingsgerät (16) nicht verwendet werden darf.

6. Das Verfahren nach Anspruch 1, das ferner die Schritte des Detektierens eines anderen physiologischen Zustands des Benutzers nach Beendigung der Benutzung des zu analysierenden Trainingsgeräts (16) durch den Benutzer umfasst.

7. Ein Sicherheits-Sicherstellungssystem eines Trainingsgeräts (16), das eine physiologische Detektionsvorrichtung (10) und ein Trainingsgerät (16) umfasst, wobei
die physiologische Detektionsvorrichtung (10) eine Datenspeichereinheit (142) aufweist, wobei die physiologische Detektionsvorrichtung (10) konfiguriert ist, um einen physiologischen Zustand eines menschlichen Körpers zu detektieren und den physiologischen Zustand in der Datenspeichereinheit (142) zu speichern; und
das Trainingsgerät (16) eine Steuervorrichtung (18) und eine Arbeitsvorrichtung (20) umfasst, wobei die Steuervorrichtung (18) elektrisch mit der physiologischen Detektionsvorrichtung (10) verbunden ist; **dadurch gekennzeichnet, dass**
die Steuervorrichtung (18) konfiguriert ist, um gemäß dem in der Datenspeichereinheit (142) gespeicherten physiologischen Zustand zu ermitteln, ob die Arbeitsvorrichtung (20) verwendet werden darf;
wobei die Steuervorrichtung (18) des Trainingsgeräts (16) konfiguriert ist, um den in der Datenspeichereinheit (142) gespeicherten physiologischen Zustand abzurufen; die Steuervorrichtung (18) konfiguriert ist, um die Arbeitsvorrichtung (20) derart zu steuern, dass sie verwendet werden darf, wenn der physiologische Zustand als ein normaler Zustand klassifiziert wird; die Steuervorrichtung (18) konfiguriert ist, um die Arbeitsvorrichtung (20) derart zu steuern, dass sie nicht verwendet werden darf, wenn der physiologische Zustand als ein unnormaler Zustand klassifiziert wird;
wobei die Steuervorrichtung (18) des Trainingsgeräts (16) eine Warneinheit (188) umfasst; die Steuervorrichtung (18) konfiguriert ist, um die Arbeitsvorrichtung (20) derart zu steuern, dass sie mit einer von der Warneinheit (188) ausgesendeten Warnung verwendet werden darf, wenn der physiologische Zustand als ein kritischer Zustand klassifiziert wird;
wobei die Steuervorrichtung (18) konfiguriert ist, um die Arbeitsvorrichtung (20) derart zu steuern, dass sie mit begrenzter Intensität verwendet werden darf, wenn der physiologische Zustand als ein kritischer Zustand klassifiziert wird.

8. Das Sicherheits-Sicherstellungssystem nach Anspruch 7, wobei die physiologische Detektionsvorrichtung (10) ein physiologisches Detektionsinstrument (12) und einen Server (14) umfasst, wobei das physiologische Detektionsinstrument (12) konfiguriert ist, um physiologische Signale eines menschlichen Körpers zu erfassen; der Server (14) mit der Steuervorrichtung (18) verbunden ist und konfiguriert ist, um den physiologischen Zustand durch Analysieren der physiologischen Signale zu erzeugen; wobei der Server (14) die Datenspeichereinheit (142) aufweist.

9. Das Sicherheits-Sicherstellungssystem nach Anspruch 8, wobei die physiologischen Signale Blutdruck, Hämoglobin-Sauerstoffsättigung oder Elektrokardiographie umfassen.

10. Das Sicherheits-Sicherstellungssystem nach Anspruch 7, wobei die physiologische Detektionsvorrichtung (10) eine erste Identifizierungseinheit (122) zum Empfangen eines Identitätscodes enthält; die Datenspeichereinheit (142) mit dem von der ersten Identifizierungseinheit (122) empfangenen Identitätscode und dem physiologischen Zustand, der dem Identitätscode entspricht, gespeichert wird; die Steuervorrichtung (18) eine zweite Identifizierungseinheit (182) zum Empfangen des Identitätscodes enthält, wobei die Steuervorrichtung (18) konfiguriert ist, um den in der Datenspeichereinheit (142) gespeicherten physiologischen Zustand gemäß dem von der zweiten Identifizierungseinheit (182) empfangenen Identitätscode abzurufen.

## Revendications

1. Procédé mis en œuvre par ordinateur visant à assurer la sécurité, appliqué à un système destiné à assurer la sécurité, comprenant un dispositif de détection physiologique (10) et une machine d'entraînement (16), où le dispositif de détection physiologique (10) présente une unité de stockage de données (142), le dispositif de détection physiologique (10) détecte l'état physiologique d'un corps humain, et sauvegarde l'état physiologique dans l'unité de stockage de données (142) ; la machine d'entraînement (16) comprenant un dispositif de commande (18) et un dispositif de travail (20), le dispositif de commande (18) est connecté électriquement au dispositif de détection physiologique (10), le procédé mis en œuvre par ordinateur visant à assurer la sécurité, comprend les étapes suivantes :
A. établir une correspondance entre un code d'identité et un état physiologique d'un utilisateur ;
B. entrer le code d'identité dans la machine d'entraînement (16) ; et
C. retrouver l'état physiologique correspondant au code d'identité entré ;
**caractérisé par** l'étape supplémentaire suivante :
D. déterminer si la machine d'entraînement (16) est autorisée à être utilisée ou non selon l'état physiologique retrouvé ;
où la machine d'entraînement (16) est autorisée à être utilisée si l'état physiologique retrouvé est classé comme étant un état normal ;
où la machine d'entraînement (16) n'est pas autorisée à être utilisée si l'état physiologique retrouvé est classé comme étant un état anormal ;
où la machine d'entraînement (16) est autorisée à être utilisée, une alerte étant envoyée si l'état physiologique est classé comme étant un état critique ;
où la machine d'entraînement (16) est autorisée à être utilisée avec une intensité limitée si l'état physiologique est classé comme étant un état critique.

2. Procédé selon la revendication 1, où l'étape A comprend les étapes suivantes :
entrer le code d'identité dans le dispositif de détection physiologique (10) ;
détecter l'état physiologique de l'utilisateur avec le dispositif de détection physiologique (10) ; et
établir une correspondance entre le code d'identité entré et l'état physiologique détecté.

3. Procédé selon la revendication 2, où le dispositif de détection physiologique (10) détecte des signaux physiologiques du corps humain ; et l'état physiologique est obtenu en analysant les signaux physiologiques.

4. Procédé selon la revendication 3, où les signaux physiologiques comprennent une tension artérielle, une saturation en hémoglobine oxygénée, ou une électrocardiographie.

5. Procédé selon la revendication 1, où dans l'étape C, s'il n'est pas possible de retrouver l'état physiologique, la machine d'entraînement (16) n'est pas autorisée à être utilisée.

6. Procédé selon la revendication 1, comprenant en outre l'étape consistant à détecter un autre état physiologique de l'utilisateur à analyser, une fois que l'utilisateur a fini d'utiliser la machine d'entraînement (16).

7. Système destiné à assurer la sécurité d'une machine d'entraînement (16), comprenant un dispositif de détection physiologique (10) et une machine d'entraînement (16),
où le dispositif de détection physiologique (10) présente une unité de stockage de données (142),
où le dispositif de détection physiologique (10) est configuré pour détecter un état physiologique d'un corps humain, et pour sauvegarder l'état physiologique dans l'unité de stockage de données (142) ; et
la machine d'entraînement (16) comprenant un dispositif de commande (18) et un dispositif de travail (20),
où le dispositif de commande (18) est connecté électriquement au dispositif de détection physiologique (10) ; **caractérisé en ce que**
le dispositif de commande (18) est configuré pour déterminer si le dispositif de travail (20) est autorisé à être utilisé selon l'état physiologique sauvegardé dans l'unité de stockage de données (142) ;
où le dispositif de commande (18) de la machine d'entraînement (16) est configuré pour retrouver l'état physiologique sauvegardé dans l'unité de stockage de données (142) ;
où le dispositif de commande (18) est configuré pour commander l'autorisation d'utilisation du dispositif de travail (20), si l'état physiologique est classé comme étant état normal ;
où le dispositif de commande (18) est configuré pour commander l'interdiction d'utilisation du dispositif de travail (20), si l'état physiologique est classé comme étant état anormal ;
où le dispositif de commande (18) de la machine d'entraînement (16) comprend une unité d'avertissement (188) ;
où le dispositif de commande (18) est configuré pour commander l'autorisation d'utilisation du dispositif de travail (20), une alerte étant envoyée par l'unité d'avertissement (188) si l'état physiologique est classé comme étant état critique ;
où le dispositif de commande (18) est configuré pour commander l'autorisation d'utilisation du dispositif de travail (20) avec une intensité limitée, si l'état physiologique est classé comme étant état critique.

8. Système destiné à assurer la sécurité selon la revendication 7,
où le dispositif de détection physiologique (10) comprend un instrument de détection physiologique (12) et un serveur (14),
où l'instrument de détection physiologique (12) est configuré pour détecter des signaux physiologiques d'un corps humain ;
où le serveur (14) est connecté au dispositif de commande (18), et est configuré pour générer l'état physiologique en analysant les signaux physiologiques ;
où le serveur (14) présente l'unité de stockage de données (142).

9. Système destiné à assurer la sécurité selon la revendication 8, où les signaux physiologiques comprennent une tension artérielle, une saturation en hémoglobine oxygénée, ou une électrocardiographie.

10. Système destiné à assurer la sécurité selon la revendication 7, où le dispositif de détection physiologique (10) comprend une première unité d'identification (122) destinée à recevoir un code d'identité ; l'unité de stockage de données (142) sauvegarde le code d'identité reçu par la première unité d'identification (122), et l'état physiologique qui correspond au code d'identité; le dispositif de commande (18) comprend une seconde unité d'identification (182) destinée à recevoir le code d'identité, le dispositif de commande (18) est configuré pour retrouver l'état physiologique sauvegardé dans l'unité de stockage de données (142) selon le code d'identité reçu par la seconde unité d'identification (182).
